# EUROPEAN PATENT APPLICATION

(11) **EP 2 612 586 A1**
(43) Date of publication of application: **10.07.2013**
(21) Application number: 11821509.4
(22) Date of filing: 03.08.2011
(51) Int. Cl.: A61B 1/00, A61B 5/07

(54) **INTERNAL INFORMATION ACQUISITION DEVICE AND INTERNAL INFORMATION ACQUISITION METHOD**

(30) Priority: 02.09.2010 JP 2010196919
(71) Applicant: OLYMPUS CORPORATION, Shibuya-ku Tokyo 151-0072 (JP)
(72) Inventor: SAKAI Youhei, Tokyo 151-0072 (JP)
(74) Representative: Winter, Brandl, Fürniss, Hübner, Röss, Kaiser, Polte - Partnerschaft
(86) International application number: PCT/JP2011/067786
(87) International publication number: WO 2012/029491

(57) **Abstract**

A capsule endoscope 20 includes a plurality of function executing sections 31 and 32 which acquire information inside a body of a subject, a battery 26 which supplies power to the plurality of function executing sections 31 and 32, a switching section 23 having a plurality of switches MS 1 and MS2 which independently control power supply from the battery 26 to the respective function executing sections 31 and 32, a magnetic field sensing section 21 which receives a control signal from outside the subject, and a power control section 22 which controls the switching section 23 according to the number of times the control signal is received by the magnetic field sensing section 21.

## Description

### Technical Field

Embodiments of the present invention relate to an in-vivo information acquiring apparatus and an in-vivo information acquiring method for acquiring information inside a body of a subject and, particularly, to an in-vivo information acquiring apparatus and an in-vivo information acquiring method for controlling power supply to a plurality of function executing sections by a control signal from outside the subject.

### Background Art

In a field of endoscopes, capsule endoscopes have recently been appearing. A capsule endoscope is introduced into a body when an examinee swallows the capsule endoscope through a mouth. Until the capsule endoscope is naturally excreted, the capsule endoscope picks up images of an interior of a body while moving inside an organ such as a stomach or a small intestine according to peristaltic motion. The capsule endoscope has a transmission unit which wirelessly transmits image data in addition to an image pickup section which picks up an image. Pieces of image data obtained through image pickup by the image pickup section are sequentially transmitted by the transmission unit and are accumulated in a memory provided in a receiver outside a body of an examinee. After observation, a doctor displays an image of an interior of a body cavity on a monitor on the basis of the pieces of image data accumulated in the memory of the receiver and performs diagnosis.

The capsule endoscope obtains driving power from, e.g., a battery built in a housing. After the capsule endoscope is introduced into a living body, drive conditions cannot be controlled.

As a solution to the problem, the applicant discloses a capsule endoscope having a control section which controls power supply by two series-connected switches in Japanese Patent Application Laid-Open Publication No. 2005-237460. In the capsule endoscope, one of an operation of driving only an image pickup section and an operation of simultaneously driving the image pickup section and a transmission unit can be controlled by a control signal from an outside.

The capsule endoscope, however, cannot drive the transmission unit alone. In other words, the control section cannot control to drive a plurality of function executing sections independently.

A capsule endoscope which has respective image pickup sections at a front portion and a rear portion of an elongated housing and performs image pickup in two directions and a capsule endoscope having various sensors such as a pH sensor and a temperature sensor have been demanded to control to individually, i.e., independently drive a plurality of functional sections.

An object of embodiments of the present invention is to provide an in-vivo information acquiring apparatus and an in-vivo information acquiring method capable of controlling to independently drive a plurality of function executing sections.

### Disclosure of Invention

### Means for Solving the Problem

An in-vivo information acquiring apparatus according to one aspect of the present invention includes a plurality of function executing sections which acquire information inside a body of a subject, a power source which supplies power to the plurality of function executing sections, a switching section having a plurality of switches which independently control power supply from the power source to each of the plurality of function executing sections, a signal receiving section which receives a control signal from outside the subject, and a power control section which controls the switching section according to a number of times that the control signal is received by the signal receiving section.

An in-vivo information acquiring method according to another aspect of the present invention includes an introduction step of introducing, into a body of a subject, an in-vivo information acquiring apparatus having a plurality of function executing sections which acquire information inside the body of the subject and a power supply control step of independently controlling power supply to the plurality of function executing sections according to a number of times that a control signal transmitted from outside the body of the subject is received.

### Brief Description of the Drawings

Fig. 1 is a configuration diagram showing a configuration of an endoscope system having a capsule endoscope according to a first embodiment;
Fig. 2 is a timing chart for explaining operation of the capsule endoscope according to the first embodiment;
Fig. 3 is a schematic diagram of a power supply system of a known capsule endoscope;
Fig. 4 is a schematic diagram of a power supply system of the capsule endoscope according to the first embodiment;
Fig. 5 is a configuration diagram showing a configuration of an endoscope system having a capsule endoscope according to a second embodiment;
Fig. 6 is a timing chart for explaining operation of the capsule endoscope according to the second embodiment; and
Fig. 7 is a configuration diagram showing a configuration of an endoscope system having a capsule endoscope according to a third embodiment.

### Best Mode for Carrying Out the Invention

### <First Embodiment>

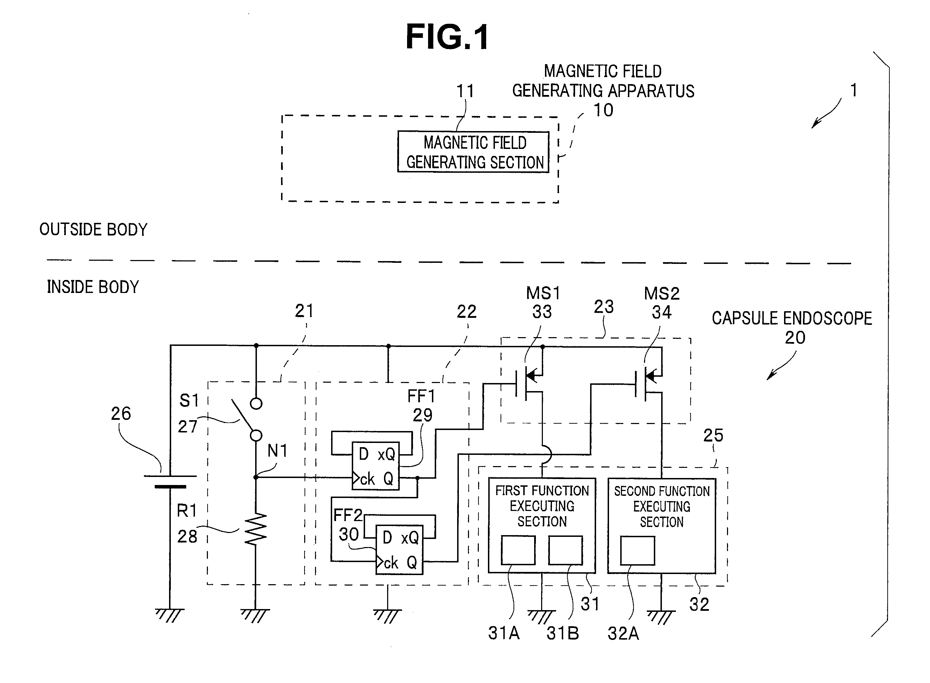

A capsule endoscope 20 which is an in-vivo information acquiring apparatus and an in-vivo information acquiring method according to a first embodiment of the present invention will be described. As shown in Fig. 1, the capsule endoscope 20 and a magnetic field generating apparatus 10 which controls operation of the capsule endoscope 20 introduced in a body from outside the body constitute an endoscope system 1 which is an in-vivo observation system. The magnetic field generating apparatus 10 has a magnetic field generating section 11 which generates a DC magnetic field pulse that is a control signal.

The capsule endoscope 20 has a function executing unit 25, a battery 26, a magnetic field sensing section 21 which is a signal receiving section, a switching section 23, and a power control section (hereinafter also referred to as a "control section") 22. The function executing unit (FES) 25 has a plurality of function executing sections (a first function executing section 31 and a second function executing section 32). The function executing unit 25 picks up an image of an interior of a body of a subject and wirelessly transmits the picked-up image to an outside.

The battery 26 is a power source which supplies driving power to the function executing unit 25. The magnetic field sensing section 21 that is a signal receiving section senses a DC magnetic field signal which is a control signal inputted from the magnetic field generating apparatus 10 and outputs an internal signal corresponding to a sensed magnetic field.

The switching section 23 has a plurality of switches (a first switch 33 and a second switch 34). The first switch 33 is a power switch MS1 which controls power supply from the battery 26 to the first function executing section 31, and the second switch 34 is a power switch MS2 which controls power supply from the battery 26 to the second function executing section 32. The first switch 33 and second switch 34 are, for example, P-MOS transistors having sources connected to the battery 26, drains connected to the function executing unit 25 that is a main functional section of the capsule endoscope 20, and gates connected to the control section 22.

The power control section 22 controls to toggle the switching section 23 according to an internal signal inputted from the magnetic field sensing section 21, i.e., the number of times the magnetic field sensing section 21 has received a control signal.

The magnetic field sensing section 21 that is a signal receiving section has a reed switch (S1) 27 and a resistor (R1) 28. In the reed switch 27, two ferromagnetic reeds are sealed in a glass tube while the ferromagnetic reeds face each other at one ends with a gap between the reeds. When a magnetic field not less than a predetermined threshold is applied from the outside to the reed switch 27, an N pole or an S pole is induced on each reed, and the two reeds are short-circuited due to a magnetic attractive force between the reeds. When the magnetic field becomes less than the threshold, the reed switch 27 is opened due to elasticity of the reeds. The resistor 28 is a pull-down resistor for setting a voltage inputted to the power control section 22 to low level when the reed switch 27 is open.

Since the reed switch 27 is opened when the magnetic field sensing section 21 does not sense a magnetic field, the magnetic field sensing section 21 outputs a signal at ground voltage (L) level to a node N1. Since the reed switch 27 falls into a short circuit condition when the magnetic field sensing section 21 senses a magnetic field, the magnetic field sensing section 21 outputs a signal at source voltage (H) level from the battery 26 to the node N1. A signal outputted to the node N1 will be referred to as an internal signal hereinafter. Since a DC magnetic field generated by the magnetic field generating section 11 is a pulse signal, an internal signal is a pulse signal at source voltage level/ground voltage level.

The control section 22 has two D flip-flop circuits 29 and 30 having respective input terminals (CK terminals), to which an internal signal is inputted. An output (a Q terminal) of the first flip-flop circuit (hereinafter also referred to as "FF1") 29 is inputted to the gate of the first switch (MS1) 33, and an output (a Q terminal) of the second flip-flop circuit (hereinafter also referred to as "FF2") 30 is inputted to the gate of the second switch (MS2) 34. That is, the plurality of switches 33 and 34 are respectively disposed in parallel between the plurality of function executing sections 31 and 32 and the battery 26.

The power control section 22 having the two D flip-flop circuits 29 and 30 can control to place the first function executing section 31 and second function executing section 32 in any one of four states. That is, the four states are (state A) in which the first function executing section is off and the second function executing section is off, (state B) in which the first function executing section is on and the second function executing section is off, (state C) in which the first function executing section is off and the second function executing section is on, and (state D) in which the first function executing section is on and the second function executing section is on. In other words, the switching section 23 independently controls power supply to the function executing section 31 and power supply to the function executing section 32.

The first function executing section 31 has an illumination section 31A which irradiates an image pickup region at the time of photographing an interior of a body of a subject and an image pickup section 31B which picks up an image of the interior of the body. The second function executing section 32 has a transmission section 32A which wirelessly transmits an image pickup signal to outside the body.

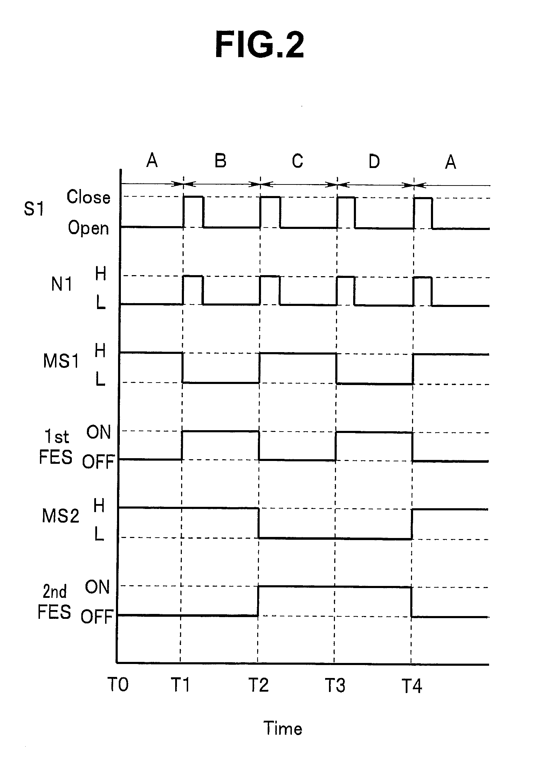

The operation of the capsule endoscope 20 will be described with reference to a timing chart in Fig. 2. Note that operation of FF1 (29) and FF2 (30) will be described as leading edge operation.

### <T0 to T1> State A: Introduction Process

The capsule endoscope 20 is introduced into a body of an examinee through deglutition. Since the reed switch (S1) 27 is open (off) at the time, the node N1 is at ground voltage level (L), Q outputs of FF1 (29) and FF2 (30) are at H level, and no current flows between the source and drain of each of MS1 (33) and MS2 (34). The first function executing section (FES1) 31 and second function executing section (FES2) 32 are thus controlled so as to be off.

### <T1 to T2> State B

When a DC magnetic field generated by the magnetic field generating section 11 of the magnetic field generating apparatus 10 is applied to the reed switch 27 of the capsule endoscope 20, and the reed switch 27 is turned on (brought into a short circuit condition). The node N1 is at source voltage level, H level, only during a period when the reed switch 27 is on. When the node N1 changes to H level, the Q output of FF1 (29) is inverted from H level to L level, and MS1 (33) is turned on. The first function executing section 31 is supplied with power from the battery 26 and is turned on (brought into an operating condition).

Although the CK terminal of FF2 (30) is inverted from H level to L level at the time, since the Q output keeps at H level, the second function executing section (FES2) 32 remains off (in a halt condition). That is, in state B, the first function executing section 31 is on while the second function executing section 31 is off.

### <T2 to T3> State C

When a second magnetic field pulse is applied to the reed switch 27, the reed switch 27 is turned on again. The Q output of FF1 (29) is inverted from L level to H level, and the first function executing section 31 is turned off. Since the CK terminal of FF2 (30) is inverted from L level to H level, and the Q output is inverted from H level to L level, the second function executing section (FES2) 32 is turned on. That is, in state C, the first function executing section 31 is off while the second function executing section 31 is on.

### <T3 to T4> State D

When a third magnetic field pulse is applied to the reed switch 27, the reed switch 27 is turned on again. Since the Q output of FF1 (29) is inverted from H level to L level at the time, the first function executing section (FES2) 31 remains on. Since the Q output of FF2 (30) keeps at L level, the second function executing section (FES2) 32 remains on. That is, in state D, the first function executing section 31 and second function executing section 31 are on.

### <From T4 on> State A

When a fourth magnetic field pulse is applied to the reed switch 27, the reed switch 27 is turned on. The Q output of FF1 (29) is inverted from L level to H level, and the first function executing section 31 is turned off. Since the CK terminal of FF2 (30) is inverted from H level to L level, and the Q output is inverted from L level to H level, the second function executing section (FES2) 32 is turned off. That is, the capsule endoscope 20 returns to state A, in which the first function executing section 31 and second function executing section (FES2) 32 are off.

The capsule endoscope 20 can be controlled to be placed in a state in which the first function executing section 31 or the second function executing section 32 operates alone or a state in which the first function executing section 31 and second function executing section 32 operate simultaneously, according to the number of times a magnetic field is applied. In other words, the capsule endoscope 20 can control to independently drive the plurality of function executing sections 31 and 32 according to a status of the switching section 23.

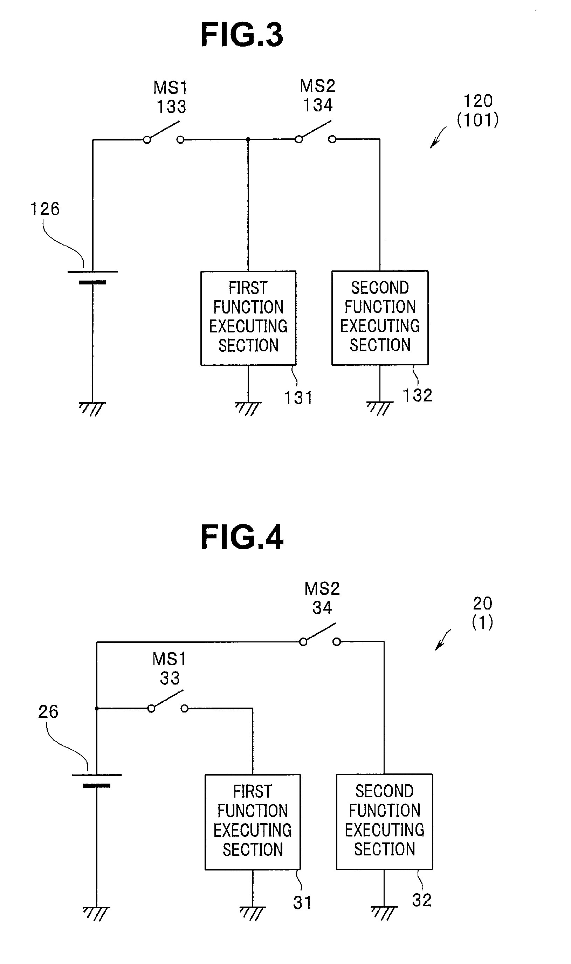

Power consumption of the capsule endoscope 20 according to the present embodiment will be compared with power consumption of a known capsule endoscope with reference to Figs. 3 and 4. Fig. 3 is a schematic diagram of a power supply system of the known capsule endoscope, and Fig. 4 is a schematic diagram of a power supply system of the capsule endoscope 20 according to the present embodiment.

As has been described above, the switch MS1 (33) and switch MS2 (34) of the switching section 23 are, for example, P-MOS transistors. A portion between the source and the drain of each switch constitutes a resistor which has a predetermined resistance RON even when the switch is conducting.

For the reason, in the known capsule endoscope shown in Fig. 3, a current to flow into a second function executing unit 25 flows from a battery 26 through a switch MS1 (33) and a switch MS2 (34). That is, power is consumed by two resistors.

In contrast, in the capsule endoscope 20 shown in Fig. 4, a current to flow into the second function executing unit 25 flows from the battery 26 through only the switch MS2 (34). That is, power is consumed by only one resistor.

Accordingly, the capsule endoscope 20 is lower in power consumption than the known capsule endoscope and can operate for a longer time.

As has been described above, the capsule endoscope 20 and the in-vivo information acquiring method according to the present embodiment independently control power supply to the plurality of function executing sections 31 and 32 and can address a variety of situations. Additionally, the capsule endoscope 20 and the in-vivo information acquiring method can operate for a long time without increasing battery capacity, due to low power consumption of resistors during switch conduction.

### <Second Embodiment>

A capsule endoscope 20A which is an in-vivo information acquiring apparatus and an in-vivo information acquiring method according to a second embodiment of the present invention will be described. The capsule endoscope 20A and in-vivo information acquiring method according to the present embodiment are similar to the capsule endoscope 20 and in-vivo information acquiring method according to the first embodiment. Same components are denoted by same reference numerals, and a description of the components will be omitted.

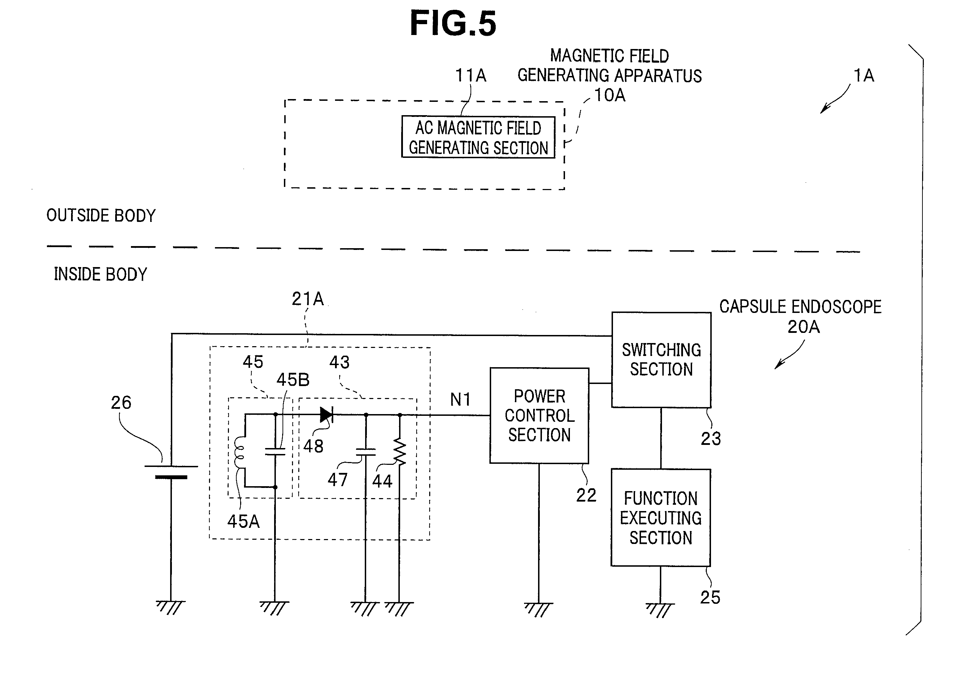

The endoscope system 1 that is an in-vivo observation system according to the first embodiment uses a DC magnetic field as an external signal. In contrast, an endoscope system 1A according to the present embodiment uses an AC magnetic field as an external signal. That is, as shown in Fig. 5, the endoscope system 1A includes an AC magnetic field generating apparatus 10A having an AC magnetic field generating section 11A which generates an AC magnetic field and the capsule endoscope 20A having an AC magnetic field sensing section 21A.

The AC magnetic field sensing section 21A of the capsule endoscope 20A in the endoscope system 1A has a power receiving section 45 which receives an AC magnetic field signal that is a control signal from the AC magnetic field generating apparatus 10A and a wave detecting section 43. An AC magnetic field signal received by the power receiving section 45 is converted to a DC signal in the wave detecting section 43 and is transmitted to a power control section 22. The power receiving section 45 has a power receiving coil 45A and a capacitor 45B. The power receiving coil 45A and capacitor 45B constitute a resonance circuit which resonates with a frequency of an AC magnetic field to be applied. The wave detecting section 43 has a diode 48 which rectifies an AC signal received by the power receiving section 45, a smoothing capacitor 47 which smoothes the rectified signal, and a resistor 44 which discharges electric charge stored in the smoothing capacitor 47. That is, the wave detecting section 43 rectifies/smoothes an AC signal and conveys a signal corresponding to an applied AC magnetic field to the control section 22.

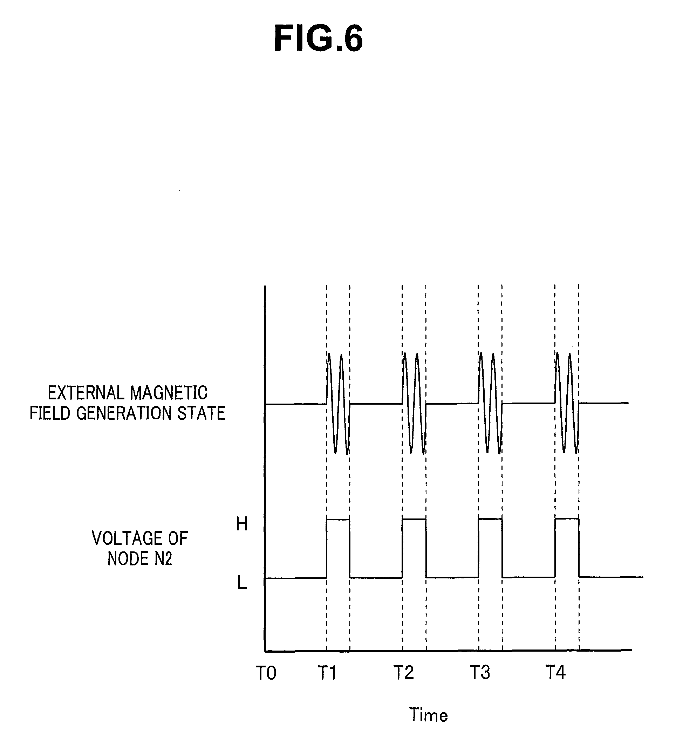

As shown in Fig. 6, only during an AC magnetic field generation period, an H-level signal is outputted to a node N2, and the H-level signal is conveyed to the power control section 22. Subsequent operation is the same as in the first embodiment.

Since the capsule endoscope 20A electromagnetically converts a received AC magnetic field to obtain a DC voltage signal, the AC magnetic field sensing section 21A does not require a power source for sensing a magnetic field.

As has been described above, the capsule endoscope 20A according to the present embodiment has the same effects as those of the capsule endoscope 20 according to the first embodiment. Additionally, since the capsule endoscope 20A uses an AC magnetic field as an external signal, the capsule endoscope 20A can be reduced in size and power consumption, as compared to the capsule endoscope 20 that senses a DC magnetic field with the reed switch 27. This is because the AC magnetic field sensing section 21A has higher sensitivity than the reed switch 27, i.e., can sense a weaker magnetic field. Moreover, the AC magnetic field sensing section 21A does not require power for sensing a magnetic field, and the capsule endoscope 20A can operate for a longer time than the capsule endoscope 20.

### <Third Embodiment>

A capsule endoscope 20B which is an in-vivo information acquiring apparatus and an in-vivo information acquiring method according to a third embodiment of the present invention will be described. The capsule endoscope 20B and in-vivo information acquiring method according to the present embodiment are similar to the capsule endoscope 20 and in-vivo information acquiring method according to the first embodiment. Same components are denoted by same reference numerals, and a description of the components will be omitted.

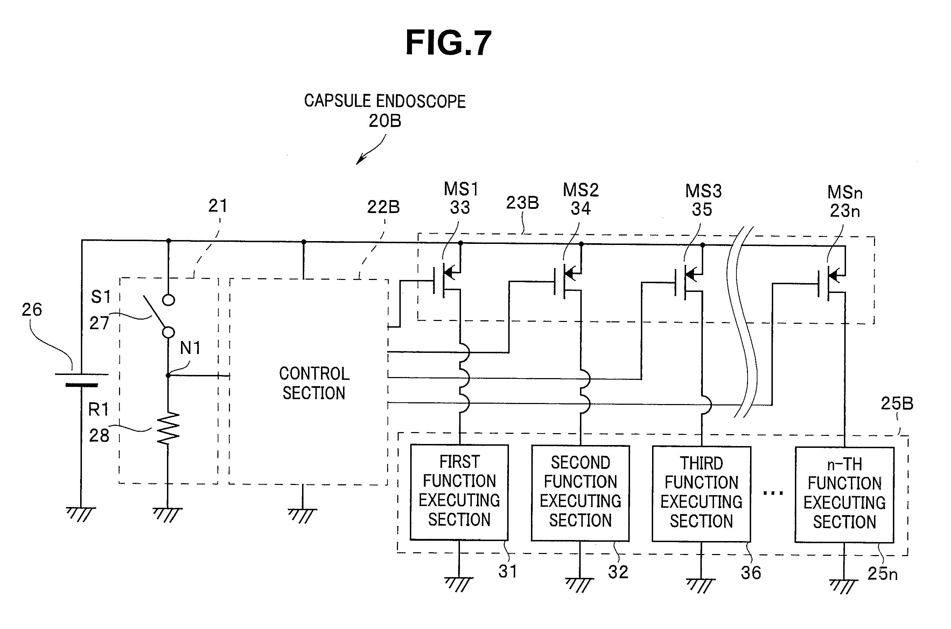

As shown in Fig. 7, a function executing unit 25B of the capsule endoscope 20B in an endoscope system 1B has not only a first function executing section 31 and a second function executing section 32 but also a third function executing section 36 to an n-th function executing section 25n. Note that n is not less than 3, preferably not less than 4. An upper limit of n depends on specifications of the capsule endoscope and is, for example, 10.

A power control section 22B has n (n ≥ 3) D flip-flop circuits, and a switching section 23B has n power switches MS1 (33) to MSn (23n) which control power supply to the respective function executing sections 31 to 25n.

For example, the power control section 22B having three D flip-flop circuits can control to place the switching section 23B in any one of eight (2³) states. That is, the power control section 22B can control to place the switching section 23B in any one of state (1) in which the first function executing section is off, the second function executing section is off, and the third function executing section is off, state (2) in which the first function executing section is on, the second function executing section is off, and the third function executing section is off, state (3) in which the first function executing section is off, the second function executing section is on, and the third function executing section is off, state (4) in which the first function executing section is off, the second function executing section is off, and the third function executing section is on, state (5) in which the first function executing section is on, the second function executing section is on, and the third function executing section is off, state (6) in which the first function executing section is on, the second function executing section is off, and the third function executing section is on, state (7) in which the first function executing section is off, the second function executing section is on, and the third function executing section is on, and state (8) in which the first function executing section is on, the second function executing section is on, and the third function executing section is on.

That is, the power control section having the n D flip-flop circuits can control to place the plurality of power switches (function executing sections) in any one of (2ⁿ) states.

For example, the first function executing section 31 has an illumination section which sheds light in an advancing direction of the capsule endoscope 20B, i.e., forward inside a body and an image pickup section which picks up an image in the direction. The second function executing section 32 has an illumination section which sheds light in a direction opposite to the direction for the first function executing section, i.e., backward inside the body and an image pickup section which picks up an image in the direction. The third function executing section 36 has a wireless transmission section which wirelessly transmits an image pickup signal obtained by picking up an image of an interior of the body to outside the body.

When the first function executing section 31 and third function executing section 36 are simultaneously operated, the capsule endoscope 20B can pick up an image in the advancing direction and wirelessly transmit the picked-up image to outside the body through the wireless transmission section. When the second function executing section 32 and third function executing section 36 are simultaneously operated, the capsule endoscope 20B can pick up an image in the direction opposite to the advancing direction (reverse direction) and wirelessly transmit the picked-up image to outside the body through the wireless transmission section. When the first to third function executing sections 31, 32, and 36 are all simultaneously operated, the capsule endoscope 20B can pick up an image in both of the advancing direction and the reverse direction and wirelessly transmit the picked-up images to outside the body through the wireless transmission section.

The capsule endoscope 20B can have, as a function executing section, for example, a temperature sensor function section which measures a temperature inside a body, a pH sensor function section which measures a pH of body fluids, or a drug delivery function section which transports a drug into the body.

Note that at least any of the plurality of function executing sections may not be an independent piece of hardware and may fulfill a function when software is executed by a CPU or the like.

That is, the capsule endoscope 20B according to the present embodiment can create a state in which one of three or more function executing sections operates alone or a state in which ones of the plurality of function executing sections operate in combination, in addition to the effects of the capsule endoscope 20 according to the first embodiment. The capsule endoscope 20B can thus address a greater variety of situations.

Additionally, the capsule endoscope 20B has n (n ≥ 3) switches. Since the capsule endoscope 20B can reduce a voltage drop resulting from on-resistance of the switches, the capsule endoscope 20B can operate for a long time without increasing battery capacity. That is, although the capsule endoscope 20B has n switches, only power corresponding to a resistor of one switch is consumed for a current to flow into each function executing section.

Note that although the above description has illustrated an example using a DC magnetic field or an AC magnetic field as an external signal, an external signal is not limited to this. Any one of an ultrasound signal and a wireless signal may be used or two or more may be used in combination.

The above description has been given with a focus on a capsule endoscope. An in-vivo observation system according to the present invention, however, can also be applied to various capsule in-vivo observation apparatuses such as a capsule medical apparatus for sampling digestive fluid and a capsule pH sensor.

The present invention is not limited to the above-described embodiments or modifications. The embodiments or modifications can be combined and can be variously changed and modified without departing from scope of the present invention.

The present application claims priority to Japanese Patent Application No. 2010-196919 filed in Japan on September 2, 2010, the disclosure of which is incorporated by reference in the specification, claims, and drawings.

## Claims

1. An in-vivo information acquiring apparatus comprising:
a plurality of function executing sections which acquire information inside a body of a subject;
a power source which supplies power to the plurality of function executing sections;
a switching section having a plurality of switches which independently control power supply from the power source to each of the plurality of function executing sections;
a signal receiving section which receives a control signal from outside the subject; and
a power control section which controls the switching section according to a number of times that the control signal is received by the signal receiving section.

2. The in-vivo information acquiring apparatus according to claim 1, wherein
each of the plurality of switches is disposed between each of the plurality of function executing sections and the power source, and
the power control section has n (n is an integer not less than 2) flip-flop circuits and controls to place the switching section in any one of 2ⁿ different states.

3. The in-vivo information acquiring apparatus according to claim 2, wherein the control signal is a magnetic field signal.

4. The in-vivo information acquiring apparatus according to claim 3, wherein
the control signal is a DC magnetic field signal, and
the signal receiving section is a reed switch which is turned on or off by the DC magnetic field signal.

5. The in-vivo information acquiring apparatus according to claim 3, wherein
the control signal is an AC magnetic field signal, and
the signal receiving section has a power receiving section and a wave detecting section, converts the received AC magnetic field signal to a DC signal, and transmits the DC signal to the power control section.

6. The in-vivo information acquiring apparatus according to claim 1, wherein the in-vivo information acquiring apparatus is a capsule endoscope.

7. An in-vivo information acquiring method comprising:
an introduction step of introducing, into a body of a subject, an in-vivo information acquiring apparatus having a plurality of function executing sections which acquire information inside the body of the subject; and
a power supply control step of independently controlling power supply to the plurality of function executing sections according to a number of times that a control signal transmitted from outside the body of the subject is received.

8. The in-vivo information acquiring method according to claim 7, wherein in the power supply control step, each of a plurality of switches which are respectively disposed between each of the plurality of function executing sections and a power source is controlled.

9. The in-vivo information acquiring method according to claim 8, wherein the control signal is a magnetic field signal.

10. The in-vivo information acquiring method according to claim 8, wherein the control signal is a DC magnetic field signal.

11. The in-vivo information acquiring method according to claim 8, wherein
the control signal is an AC magnetic field signal, and
the AC magnetic field signal received by the signal receiving section is converted to a DC signal.

12. The in-vivo information acquiring method according to claim 7, wherein the in-vivo information acquiring apparatus is a capsule endoscope.
